# EUROPEAN PATENT APPLICATION

(11) **EP 3 418 393 A1**
(43) Date of publication of application: **26.12.2018**
(21) Application number: 17753458.3
(22) Date of filing: 15.02.2017
(51) Int. Cl.: C12Q 1/68, G01N 33/533

(54) **SINGLE-STRANDED NUCLEIC ACID FOR DETECTING NUCLEIC ACID OR PROTEIN IN REAL TIME AND DETECTION METHOD USING SAME**

(30) Priority: 15.02.2016 KR 20160017359; 14.02.2017 KR 20170020238
(71) Applicant: Nuribio Co., Ltd., Suwon-si, Gyeonggi-do 16229 (KR)
(72) Inventor: NAM, Young Hyean, Suwon-si Gyeonggi-do 16243 (KR)
(74) Representative: Andrews, Robert
(86) International application number: PCT/KR2017/001632
(87) International publication number: WO 2017/142297

(57) **Abstract**

The present invention relates to a promer that has a structure of X-Y-Z and that comprises a detectable marker attached to both ends or the inside thereof and also that is used as a primer and a probe during real-time detection of nucleic acid or protein, and to a method for real-time detection of nucleic acid, comprising amplifying a nucleic acid to be detected using the promer, and then measuring the amount of fragments of the promer cleaved, or to a method for real-time detection of protein. The method for detection of nucleic acid or protein according to the present invention uses a small amount of oligos compared to a conventional detection method, does not require a separate probe for real-time detection, and thus can achieve real-time detection of the nucleic acid or protein to be detected in a cost-effective and simple manner. Furthermore, mutations in the Y region can be detected through amplification after cleavage of the Y region of the promer, and multiplex detection of nucleic acids or proteins larger than the number of fluorescent labels attached to the promer is possible. Thus, the present invention can be effectively used for diagnosis of various diseases and for prognostic diagnosis.

<110>     Nuribio Co., Ltd.

<120>     Promer for Real-Time Detection of Nucleic Acid or Protein and
          Method of Detecting Nucleic Acid or Protein

<130>     FP-17-0102

<150>     KR 10-2016-0017359
<151>     2016-02-15

<150>     KR 10-2017-0020238
<151>     2017-02-14

<160>     23

<170>     KoPatentIn 3.0

<210>     1
<211>     20
<212>     DNA_RNA
<213>     Artificial Sequence

<220>
<223>     Human CK18 Forward promer


<220>
<223>     RNA is located in 17th and 18th of sequence.


<400>     1
atcttggtga tgccttggac                                                   20


<210>     2
<211>     20
<212>     DNA
<213>     Artificial Sequence

<220>
<223>     Human CK18 Reverse primer


<400>     2
cctgcttctg ctggcttaat                                                   20


<210>     3
<211>     20
<212>     DNA
<213>     Artificial Sequence

<220>
<223>     Human CK19 forward primer


<400>     3
gtcacagctg agcatgaaag                                                   20


<210>     4
<211>     20
<212>     DNA_RNA

<213>    Artificial Sequence

<220>
<223>    Human CK19 Reverse promer


<220>
<223>    RNA is located in 17th and 18th of sequence.


<400>    4
tcactatcag ctcgcacatc                                                    20


<210>    5
<211>    20
<212>    DNA_RNA
<213>    Artificial Sequence

<220>
<223>    Human GAPDH Forward promer


<220>
<223>    RNA is located in 17th and 18th of sequence.


<400>    5
aaggtgaagg tcggagucaa                                                    20


<210>    6
<211>    20
<212>    DNA
<213>    Artificial Sequence

<220>
<223>    Human GAPDH Reverse primer


<400>    6
aatgaagggg tcattgatgg                                                    20


<210>    7
<211>    20
<212>    DNA
<213>    Artificial Sequence

<220>
<223>    Human CK18 Forward primer


<400>    7
atcttggtga tgccttggac                                                    20


<210>    8
<211>    20
<212>    DNA
<213>    Artificial Sequence

<220>

<223>    Human CK19 Reverse primer

<400>    8
tcactatcag ctcgcacatc                                                    20

<210>    9
<211>    20
<212>    DNA
<213>    Artificial Sequence

<220>
<223>    Human GAPDH Forward primer

<400>    9
aaggtgaagg tcggagtcaa                                                    20

<210>    10
<211>    22
<212>    DNA_RNA
<213>    Artificial Sequence

<220>
<223>    G12D Forward promer

<220>
<223>    RNA is located in 20th of sequence.

<400>    10
cttgtggtag ttggagctga tg                                                 22

<210>    11
<211>    23
<212>    DNA_RNA
<213>    Artificial Sequence

<220>
<223>    G12V Forward promer

<220>
<223>    RNA is located in 21th of sequence.

<400>    11
acttgtggta gttggagctg atg                                                23

<210>    12
<211>    25
<212>    DNA
<213>    Artificial Sequence

<220>
<223>    G12D and G12V Uni-reverser primer

<400>    12
catattcgtc cacaaaatga ttctg                                              25


<210>    13
<211>    810
<212>    DNA
<213>    Artificial Sequence

<220>
<223>    a nucleotid located in 241 to 1030 at OPN1MW T529C mutant gene


<400>    13
ttcaagaagc tgcgccaccc gctgaactgg atcctggtga acctggcggt cgctgacctg        60

gcagagaccg tcatcgccag cactatcagc gttgtgaacc aggtctatgg ctacttcgtg       120

ctgggccacc ctatgtgtgt cctggagggc tacaccgtct ccctgtgtgg gatcacaggt       180

ctctggtctc tggccatcat ttcctgggag agatggatgg tggtctgcaa gccctttggc       240

aatgtgagat ttgatgccaa gctggccatc gtgggcattg ccttctcccg gatctgggct       300

gctgtgtgga cagccccgcc catctttggt tggagcaggt actggcccca cggcctgaag       360

acttcatgcg gcccagacgt gttcagcggc agctcgtacc ccggggtgca gtcttacatg       420

attgtcctca tggtcacctg ctgcatcacc ccactcagca tcatcgtgct ctgctacctc       480

caagtgtggc tggccatccg agcggtggca aagcagcaga aagagtctga atccacccag       540

aaggcagaga aggaagtgac gcgcatggtg gtggtgatgg tcctggcatt ctgcttctgc       600

tggggaccat acgccttctt cgcatgcttt gctgctgcca accctggcta ccccttccac       660

cctttgatgg ctgccctgcc ggccttcttt gccaaaagtg ccactatcta caaccccgtt       720

atctatgtct ttatgaaccg gcagtttcga aactgcatct tgcagctttt cgggaagaag       780

gttgacgatg gctctgaact ctccagcgcc                                        810


<210>    14
<211>    20
<212>    DNA_RNA
<213>    Artificial Sequence

<220>
<223>    529CA1 Promer


<220>
<223>    RNA is located in 18th of sequence.


<400>    14
tgggcattgc cttctcccgg                                                    20


<210>    15
<211>    12
<212>    DNA_RNA

<213>    Artificial Sequence

<220>
<223>    529CA2 Promer


<220>
<223>    RNA is located in 10th of sequence.


<400>    15
gccttctccc gg                                                            12


<210>    16
<211>    34
<212>    DNA_RNA
<213>    Artificial Sequence

<220>
<223>    529CA3 Promer


<220>
<223>    RNA is located in 32th of sequence.


<400>    16
caagctggcc atcgtgggca ttgccttctc ccgg                                    34


<210>    17
<211>    28
<212>    DNA_RNA
<213>    Artificial Sequence

<220>
<223>    529CA4 Promer


<220>
<223>    RNA is located in 18th of sequence.


<400>    17
tgggcattgc cttctcccgg atctgggc                                           28


<210>    18
<211>    35
<212>    DNA_RNA
<213>    Artificial Sequence

<220>
<223>    529CA5 Promer


<220>
<223>    RNA is located in 18th of sequence.


<400>    18
tgggcattgc cttctcccgg atctgggctg ctgtg                                   35

<210>    19
<211>    20
<212>    DNA
<213>    Artificial Sequence

<220>
<223>    529CA Reverse primer


<400>    19
gtacctgctc caaccaaaga                                                    20


<210>    20
<211>    22
<212>    DNA_RNA
<213>    Artificial Sequence

<220>
<223>    G12D Forward Promer rY Type


<220>
<223>    RNA is located in 21th of sequence.


<400>    20
acttgtggta gttggagctg at                                                 22


<210>    21
<211>    22
<212>    DNA_RNA
<213>    Artificial Sequence

<220>
<223>    G12D Forward Promer rYY Type


<220>
<223>    RNA is located in 20th of sequence.


<400>    21
cttgtggtag ttggagctga tg                                                 22


<210>    22
<211>    23
<212>    DNA_RNA
<213>    Artificial Sequence

<220>
<223>    G12V Forward Promer rY Type


<220>
<223>    RNA is located in 22th of sequence.


<400>    22

aacttgtggt agttggagct gut                                              23

<210>    23
<211>    23
<212>    DNA_RNA
<213>    Artificial Sequence

<220>
<223>    G12V Forward Promer rYY Type

<220>
<223>    RNA is located in 21th of sequence.

<400>    23
acttgtggta gttggagctg utg                                              23

## Description

### [Technical Field]

The present invention relates to a promer which can be used as both a primer and a probe for real-time detection of nucleic acid or protein, and a method of detecting nucleic acid or protein in real time by use of the promer.

### [Background Art]

As is known in the art, methods that are widely used for real-time detection of nucleic acid or protein include microarray assays, real-time polymerase chain reaction (RT-PCR), quantitative real-time polymerase chain reaction (qRT-PCR), and isothermal amplification methods such as NASBA, RCA, TDMA or the like.

Such methods for real-time detection of nucleic acid or protein require forward/reverse primers and either intercalator type dsDNA-binding agents (such as SYBR) or the probe type such as Taqman probe, molecular beacon, MGB probe, CataCleave probe or the like for real-time detection. Such detection methods have several limitations. As one example, for analysis of short-length nucleic acid such as miRNA, it is required to form loop RT primers or poly(A) for cDNA synthesis and to extend an additional oligonucleotide following poly(A). Thus, analysis of short-length nucleic acid such as miRNA requires a complex procedure, increased additional costs and a long detection time. In addition, the methods have disadvantages in that one miRNA to be detected can be labeled with only one fluorescent label and in that, in systems that are currently used to detect fluorescent labels, the number of fluorescent channels that can be analyzed at the same time is generally limited to 4-7, and for this reason, the same operation should be repeated twice or more to analyze 8 or more miRNAs.

As another example that is limited by existing method, an oligonucleotide that is amplified for analysis of a target nucleic acid is required to have a length of at least 60-70 bp in view of the lengths of forward/reverse primers and a probe. In this case, at least three specific sequences are required.

As still another example, to measure the mutation of a nucleotide at a single position, such as SNP (single nucleotide polymorphism), the binding affinity at a specific mutation position of a primer or a probe should be increased using a technology such as PNA (peptide nucleic acid) or LNA (locked nucleic acid) technology for endpoint genotyping. This makes primer and probe construction difficult and requires much time and cost.

On the other hand, U.S. Patent No. 5,763,181 discloses a method for detecting nucleic acids or proteins in real time. The CataCleave technique differs from the Taq-Man probe in that the cleavage of the probe is performed by a secondary enzyme that lacks polymerase activity. The CataCleave probe has a sequence within the target molecule of the endonuclease such as, for example, a restriction enzyme or RNase. In one embodiment, the CataCleave probe has a chimeric structure in which the 5 'and 3' ends of the probe are composed of DNA and the cleavage site is composed of RNA. The DNA sequence portion of the probe is labeled with a FRET pair at either end or inside. The PCR reaction involves an RNase H enzyme capable of specifically cleaving the RNA sequence portion of the RNA-DNA double strand. After cleavage, all cleaved probes are dissociated from the target amplicon at the reaction temperature and dispersed in the reaction solution. As donors and receptors are separated, FRET is changed in the same way as the Taq-Man probe and donor release can be monitored. The cleavage and dissociation will reproduce the site for additional CataCleave probe binding. In this way, a single amplicon can be used to repeat the probe cleavage multiple times as a target, until the primer is stretched through the CataCleave probe binding site.

However, the real-time detection method using the Catacleave probe disclosed in the above-mentioned US patent has some problems.

First, background fluorescence values are continuously increased due to repetitive cleavage of the Catacleave probe during detection. Accordingly, it is difficult to accurately measure the Ct value, and a separate operation is required to compensate the Ct value.

Second, Catacleave probe requires at least 60-70 bp of amplification interval.

Third, it is difficult to analyze SNP using the Catacleave probe. In other words, Catacleave probe is difficult to apply to existing endpoint genotyping, and the method of directly hybridizing to the point mutation site is less accurate.

### [Disclosure]

### [Technical Problem]

Accordingly, in order to overcome the above problems and the limitations of the prior art, it is an object of the present invention to provide a promer which is used as a primer and a probe for real-time detection of nucleic acid or protein.

Another object of the present invention is to provide a method for real-time detection of RNA using the promer.

Still another object of the present invention is to provide a method for real-time detection of DNA using the promer.

Still another object of the present invention is to provide a method for real-time detection of protein using the promer.

### [Technical Solution]

In one aspect, the present invention provides a promer which is used as a primer and a probe for real-time detection of nucleic acid or protein.

Accordingly, the present inventors have studied to develop a method for real-time detection of nucleic acid or protein, which has excellent accuracy, reproducibility and resolution and requires, *inter alia,* a short amplification length and time. As a result, the present inventors have developed a promer that has a structure of X-Y-Z and that can be used as both a primer and a probe in a process for detecting nucleic acid or protein, and have found that, when the amount of fragments of the promer cleaved in a process for amplifying nucleic acid using the promer or a process of hybridization with protein using the promer is measured, the nucleic acid or protein to be detected can be accurately and quickly detected, thereby completing the present invention.

In the present invention, the promer may have a structure of X-Y-Z and comprise one or more detectable markers attached to both ends or the inside thereof. Herein, the positions of the attached detectable markers are not limited to particular positions and may be any positions at which the detectable markers are separated when Y of the promer is cleaved by a specific enzyme.

Furthermore, the promer may form a complex by binding to a specific region of the target nucleic acid or target protein to be detected in real time. When Y region is cleaved by a specific enzyme, the Y and Z regions of the promer may be separated from the specific region of the target nucleic acid or target protein, but the X region is not separated and retains the complex and is used as a primer for amplification.

The nucleic acid structure developed by the present inventors was termed "promer" in the present invention. Hereinafter, unless otherwise specified, the term "promer" refers to the nucleic acid structure that can be used as both a primer and a probe.

The promer of the present invention has the structure of X-Y-Z, and each of X, Y, and Z may have various numbers of nucleotides.

In one example, the X region of the promer is a DNA or RNA consisting of 1-60 nucleotides, preferably 1-30 nucleotides, more preferably 2-30 nucleotides, even more preferably 3-30 nucleotides, still even more preferably 4-30 nucleotides, still even more preferably 5-30 nucleotides, still even more preferably 6-30 nucleotides, still even more preferably 7-30 nucleotides, still even more preferably 8-30 nucleotides, still even more preferably 10-30 nucleotides. If the X region of the promer comprises more than 60 nucleotides, there will be a problem in that a nonspecific reaction occurs during real-time detection of nucleic acid or protein.

In one example, the Y region of the promer is a DNA or RNA consisting of 1-10 nucleotides, preferably 1-9 nucleotides, more preferably 1-8 nucleotides, even more preferably 1-7 nucleotides, still even more preferably 1-6 nucleotides, still even more preferably 1-5 nucleotides, still even more preferably 1-4 nucleotides, still even more preferably 1-3 nucleotides, still even more preferably 1-2 nucleotides. When the Z region of the promer comprises no nucleotide sequence, the Y region of the promer consists of at least 3 nucleotides, preferably 3-10 nucleotides, more preferably 3-9 nucleotides, even more preferably 3-8 nucleotides, still even more preferably 3-7 nucleotides, still even more preferably 3-6 nucleotides, still even more preferably 3-5 nucleotides, still even more preferably 3-4 nucleotides. When the Z region of the promer comprises one nucleotide sequence, the Y region of the promer consists of at least 2 nucleotides, preferably 2-10 nucleotides, more preferably 2-9 nucleotides, even more preferably 2-8 nucleotides, still even more preferably 2-7 nucleotides, still even more preferably 2-6 nucleotides, still even more preferably 2-5 nucleotides, still even more preferably 2-4 nucleotides, still even more preferably 2-3 nucleotides. If the number of nucleotides forming the Y region of the promer is out of the above-described range, there will be a problem in that a nonspecific reaction occurs during real-time detection of nucleic acid or protein to reduce sensitivity.

In one example, the Z region of the promer is a DNA or RNA consisting of 0-10 nucleotides, preferably 1-10 nucleotides, more preferably 2-10 nucleotides. If the number of nucleotides forming the Z region is out of the above-described range, there will be a problem in that the Z region bound to the target nucleic acid or protein is not separated from the target nucleic acid or protein after cleavage of the Y region, and thus the nucleic acid or protein cannot be detected.

At this time, when any one of X, Y and Z of the promer is DNA, one or more of the other two are RNA. Preferably, X, Y and Z are DNA, RNA and DNA, respectively, or RNA, DNA and RNA, respectively.

In one specific example, when the X region is DNA, any one of Y and Z is RNA. For example, Y is RNA, and Z is DNA. Alternatively, Y is DNA, and Z is RNA. Herein, the number of DNA and RNA may be more than 0, and is as defined above for X, Y and Z.

In another specific example, when X is RNA, any one of Y and Z is RNA. For example, Y is RNA, and Z is DNA. Alternatively, Y is DNA, and Z is RNA. Herein, the number of DNA and RNA may be more than 0, and is as defined above for X, Y and Z.

In still another specific example, when Z is null, any one of X and Y is DNA, and the other one is RNA. Herein, the number of DNA and RNA may be more than 1, and is as defined above for X and Y.

Furthermore, X, Y and Z of the promer may be synthesized so as to be wholly or partially methylated to prevent nonspecific cleavage.

In the present invention, the term "nucleic acid" refers to DNA or RNA to be detected in real time in a sample.

In the present invention, the detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

When a fluorescent pair is used as the detectable marker in the present invention, the fluorescent label and the quencher may be located in the X or Z region or located in the Y region, and the location thereof is not limited to any location. In one example, the fluorescent label may be located in the X region, and the quencher may be located in the Y or Z region.

The promer of the present invention may be used as the following primer or probe in detection of nucleic acid or protein: i) an RT primer for synthesizing cDNA from RNA among nucleic acids; ii) a forward primer for amplifying cDNA synthesized from DNA or RNA; iii) a reverse primer for amplifying cDNA synthesized from DNA or RNA; iv) a forward primer and a reverse primer for amplifying cDNA synthesized from DNA or RNA; or v) a probe for detecting nucleic acid (DNA or RNA) or protein in real time.

In one specific embodiment, the promer of the present invention may be used as: i) an RT primer for synthesizing cDNA from RNA (including small RNA such as miRNA), and a probe; or ii) a forward primer for amplifying synthesized cDNA, and a probe; or iii) a reverse primer for amplifying synthesized cDNA, and a probe; or iv) forward and reverse primers for amplifying synthesized cDNA, and a probe.

Particularly, when the promer of the present invention is used either as an RT primer for synthesizing cDNA from RNA (including small RNA such as miRNA), as a forward primer and a probe, or as a reverse primer and a probe, a process for synthesizing a looped RT primer for cDNA synthesis or forming poly(A) is not required, and the promer can synthesize cDNA by hybridization with the RNA to be detected and can achieve the amplification and real-time detection of the RNA (including small RNA such as miRNA) to be detected.

In another specific embodiment, the promer of the present invention may be used as the following primer and probe to detect a specific DNA in a sample: i) a forward primer and a probe; ii) a reverse primer and a probe; or iii) a forward primer, a reverse primer and a probe.

In such specific embodiments, the Y region of the promer of the present invention may be cleaved by an enzyme that cleaves the Y region, and then the Y and Z regions may be separated from the template, and the X region may be used as a primer by retaining a complex with the target nucleic acid to synthesize and amplify nucleic acid. Furthermore, because only the Y region of the promer is cleaved by an enzyme that cleaves the Y region, the promer of the present invention can more accurately detect the nucleic acid or protein to be detected, compared to a conventional probe that is degraded by DNA polymerase.

Particularly, where a fluorescent label or a quencher is bound to 3' end of the Y or Z region in the promer of the present invention, amplification of the target nucleic acid or a specific nucleic acid of the target protein by polymerase can be prevented, unless the Y region is cleaved. Thus, the promer of the present invention can accurately detect mutations such as point mutation, insert mutation or deletion mutation.

Accordingly, when the promer of the present invention is used to detect nucleic acid or protein, the analysis time and cost can be reduced, and the nucleic acid or protein to be detected can be more accurately and specifically detected compared to a conventional method. Thus, the promer of the present invention may be used as a kit for real-time detection of nucleic acid or protein.

Where the promer of the present invention is used as a kit for real-time detection of nucleic acid or protein, the kit preferably further comprises, in addition to the promer of the present invention, an enzyme capable of cleaving the Y region of the promer.

In the present invention, the enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABSC nuclease. When the Y region is RNA, the enzyme capable of cleaving the Y region is preferably ribonuclease (RNase), specifically RNase II, RNase III, RNase IV, RNase H, or RNase T₂.

Where the promer of the present invention is used as a kit for real-time detection of nucleic acid or protein, the kit may further comprise, in addition to the promer of the present invention and the enzyme capable of cleaving the Y region of the promer, reagents required for amplification of DNA.

The reagents required for amplification include, for example, suitable amounts of DNA polymerase (e.g., thermostable DNA polymerase derived from *Thermus aquatiucs* (Taq), *Thermus thermophilus* (Tth), *Thermus filiformis, Thermis flavus, Thermococcus literalis* or *Phyrococcus furiosis* (Pfu)), DNA polymerase cofactor (Mg²⁺), buffer, dNTPs (dATP, dCTP, dGTP and dTTP) and water (dH₂O). In addition, the buffer includes, but is not limited to, suitable amounts of Triton X-100, dimethylsufoxide (DMSO), Tween 20, nonidet P40, PEG 6000, formamide and bovine serum albumin (BSA).

In another aspect, the present invention provides a method for real-time detection of RNA using the promer of the present invention.

The method may comprise the steps of:
(a) extracting RNA from a sample; (b) adding an RT primer or the promer of the present invention to the RNA extracted in step (a), and synthesizing cDNA; (c) adding a kit comprising the promer of the present invention, and/or a forward primer or reverse primer having a nucleotide sequence complementary to the cDNA, to the cDNA synthesized in step (b), and amplifying the cDNA by extension; and (d) measuring the amount of fragments of the promer cleaved in step (c).

Each step of the method for real-time detection of RNA according to the present invention will now be described.

Step (a) is a step of extracting RNA from a sample.

In the present invention, the sample may be either a biological sample, or RNA or a fragment thereof, isolated from the biological sample. Specifically, the sample may be any one or more selected from the group consisting of blood, saliva, urine, feces, tissue, cell and biopsy samples, or may also be RNA or a fragment thereof, isolated from a stored biological sample, but is not necessarily limited thereto.

The stored biological sample may be one stored by any conventional method known in the art. The sample may be one stored for more than 1 week or more than 1 year, for example, 1 to 10 years. Alternatively, the sample may be one derived from freeze-stored tissue or from formalin-fixed tissue stored at room temperature.

In the present invention, extraction of RNA from the sample may be performed using various methods known in the art. In one example, it may be performed using trizol or Triton X-100.

Step (b) is a step of synthesizing cDNA from RNA.

Specifically, step (b) in the method of the present invention is a step of adding an RT primer to the RNA, extracted in step (a), to synthesize cDNA.

In the present invention, the RT primer is a nucleic acid consisting of 5-30 nucleotides, preferably 10-30 nucleotides, which can complementarily bind to a portion of the nucleotide sequence of the RNA to be detected. Herein, the RT primer may be either an RT primer generally known in the art, or the promer of the present invention.

In the present invention, where the promer of the present invention is used as the RT primer, the promer may comprise a nucleotide sequence that can complementarily bind to a portion of the nucleotide sequence of the nucleic acid to be detected.

In the present invention, synthesis of the cDNA may be performed various methods known in the art. In one example, it may be performed using the RNA, obtained from the sample, as a template, reverse transcriptase and DNA polymerase. On the other hand, where the above-described promer is not used as the RT primer, RNA analysis using the promer is possible, even when cDNA is synthesized using a poly(A) tail, a specific primer and a random primer.

Step (c) is a step of amplifying cDNA.

Specifically, step (c) of the method of the present invention is a step of adding a kit comprising the promer of the present invention, and/or a forward primer or reverse primer having a nucleotide sequence complementary to the cDNA, to the cDNA synthesized in step (b), and amplifying the cDNA by extension.

The kit comprising the promer refers to a kit comprising the promer of the present invention and an enzyme capable of cleaving the Y region of the promer.

The enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABSC nuclease. When the Y region is RNA, the enzyme capable of cleaving the Y region is preferably ribonuclease (RNase), specifically RNase II, RNase III, RNase IV, RNase H, or RNase T₂.

In the present invention, the promer comprises a nucleotide sequence that can complementarily bind to a portion of the nucleotide sequence of the cDNA synthesized in step (b). To amplify the cDNA, the promer may be used as: i) a forward primer and a probe; or ii) a reverse primer or a probe; or iii) a forward primer, a reverse primer and a probe. Furthermore, the promer of the present invention has a structure of X-Y-Z and comprises one or more detectable markers attached to both ends or the inside thereof. When the Y region of the promer is cleaved by a specific enzyme, the Y and Z regions are separated from the template, and the X region serves as a primer without separation from the template. The structure of such promer is as described above.

The above detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

In one specific example, where the promer of the present invention is to be used as a forward primer and a probe or as a reverse primer and a probe, the promer may comprise a nucleotide sequence capable of complementarily binding to a portion of the nucleotide sequence of the cDNA synthesized in step (b).

In another specific example, where the promer of the present invention is used as a forward primer and a probe or as a reverse primer or a probe, a separate reverse primer or forward primer may be used in addition to the promer. Herein, the separate reverse primer or forward primer is a DNA that can complementarily bind to the cDNA synthesized in step (b) and that comprises 5-30 nucleotides, preferably 10-30 nucleotides. Herein, the reverse primer may be the same as the RT primer used in step (b).

In still another specific example, where the promer of the present invention is used as a forward primer, a reverse primer and a probe, the promer that is used as the reverse primer may be the same as the promer used in step (b), and the detectable markers attached to both ends or the inside of the promer may be the same as or different from those of the promer used in step b).

In the present invention, amplification of the cDNA is performed at an isothermal temperature at which the cDNA is annealed with (A) the promer of the present invention, which is used as a forward primer and a probe or as a reverse primer and a probe, and a forward primer or reverse primer, which is generally used in the art, or (B) the promer of the present invention, which is used as a forward primer, a reverse primer and a probe, and at which the activity of the enzyme used is not substantially inhibited. As used herein, the term "isothermal temperature" means that there is no thermal cycling, and the term does not necessarily mean physically equivalent temperature.

In one specific example, the isothermal temperature at which amplification in the present invention is performed may be 40°C to 80°C, preferably 55°C to 75°C, more preferably 60°C to 75°C.

In the present invention, amplification of the cDNA may be performed by one method selected from the group consisting of polymerase chain reaction, rolling circle amplification, strand displacement amplification, and nucleic acid sequence-based amplification, but is not necessarily limited thereto.

In the present invention, amplification of the cDNA may be performed using reagents required for amplification, in addition to a kit comprising the promer of the present invention. The reagents required for amplification may include, for example, suitable amounts of DNA polymerase (e.g., thermostable DNA polymerase derived from *Thermus aquatiucs* (Taq), *Thermus thermophilus* (Tth), *Thermus filiformis, Thermis flavus, Thermococcus literalis* or *Phyrococcus furiosis* (Pfu)), DNA polymerase cofactor (Mg²⁺), buffer, dNTPs (dATP, dCTP, dGTP and dTTP) and water (dH₂O). In addition, the buffer may include, but are not limited to, suitable amounts of Triton X-100, dimethylsufoxide (DMSO), Tween 20, nonidet P40, PEG 6000, formamide and bovine serum albumin (BSA).

Step (d) is a step of measuring the amount of fragments of the primer cleaved by the enzyme capable of cleaving the Y region of the promer.

In the present invention, measurement of the amount of fragments of the promer may be performed using various detection methods. Specifically, the amount of fragments of the promer cleaved according to the present invention is preferably measured after completion of RT-PCR or amplification, and may be determined by measuring a change in fluorescence intensity or measuring chemiluminescence.

Measurement of the change in fluorescence intensity or chemiluminescence may be performed using any measurement system capable of detecting a fluorescent label, known in the art. For example, the measurement may be performed using a real-time PCR machine, a TRIAD multimode detector, a Wallac/Victor fluorescence plate reader, a Perkin-Elmer LB50B luminescence spectrometer, LightCycler 96, Applied Biosystems 7500, or Biorad CFX96 real-time PCR thermocycler, but is not limited thereto.

The method for measurement and detection of the amount of fragments of the promer cleaved according to the present invention may vary depending on the kind of label or detectable marker introduced into the promer or a reaction solution.

For example, where the promer, in which a fluorescent label is attached to the end of X region and a quencher is attached to the end of Z region, hybridized with the target nucleic acid, the fluorescence of the fluorescent label attached to the end of X region is greatly reduced by the quencher attached to the end of Z region before the Y and Z regions are cleaved by an enzyme capable of cleaving the Y region. However, when the X region is not separated by the enzyme capable of cleaving the Y region, and only the Y and Z regions are separated from the target nucleic acid and dispersed in the reaction solution, the fluorescence of the fluorescent label attached to the end of X region is increased without being affected by the quencher attached to the end of Z region. At this time, it is possible to detect the target nucleic acid in real time by measuring the increased fluorescence emission of the fluorescent label attached to the end of X region using the above-described apparatus.

In one specific example, cDNA was synthesized using an RT primer that complementarily binds to small RNA, and then the cDNA was amplified using the promer of the present invention, which complementarily binds to the cDNA and has FAM attached thereto, as a forward primer or a reverse primer. As a result, it was shown that an amplification curve appeared during amplification of the cDNA, unlike the case of a cDNA amplified using a conventional forward primer to which a detectable marker was not attached.

In still another aspect, the present invention provides a method for real-time detection of DNA using the promer of the present invention.

The method may comprise the steps of:
(a) extracting DNA from a sample; (b) adding a kit comprising the promer of the present invention, and/or a forward or reverse primer having a nucleotide sequence complementary to the DNA, extracted in step (a), and amplifying the DNA by extension; and (c) measuring the amount of fragments of the promer cleaved through step (b).

Each step of the method for real-time detection of DNA according to the present invention will now be described.

Step (a) is a step of extracting DNA from a sample.

In the present invention, the sample may be either a biological sample, or DNA or a fragment thereof, isolated from the biological sample. Specifically, the sample may be any one or more selected from the group consisting of blood, saliva, urine, feces, tissue, cell and biopsy samples, or may also be DNA or a fragment thereof, isolated from a stored biological sample, but is not necessarily limited thereto.

The stored biological sample may be stored by any conventional method known in the art. The sample may be one stored for more than 1 year, for example, 1 to 10 years. Alternatively, the sample may be one derived from freeze-stored tissue or from formalin-fixed tissue stored at room temperature.

In the present invention, extraction of DNA from the sample may be performed using various methods known in the art. In one example, it may be performed using chloroform or ethanol.

Step (b) is a step of amplifying DNA.

Specifically, step (b) of the method of the present invention is a step of adding a kit comprising the promer of the present invention, and/or a forward or reverse primer having a nucleotide sequence complementary to the DNA, extracted in step (a), and amplifying the DNA by extension.

In the present invention, the kit comprising the promer of the present invention refers to a kit comprising the promer of the present invention and an enzyme capable of cleaving the Y region of the promer.

In the present invention, the enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABSC nuclease. When the Y region is RNA, the enzyme capable of cleaving the Y region is preferably ribonuclease (RNase), specifically RNase II, RNase III, RNase IV, RNase H, or RNase T₂.

As mentioned above, the promer comprises a nucleotide sequence that can complementarily bind to a portion of the nucleotide sequence of the DNA obtained in step (a). To amplify the DNA, the promer may be used as: i) a forward primer and a probe; or ii) a reverse primer and a probe; or iii) a forward primer, a reverse primer and a probe. Furthermore, the promer of the present invention has a structure of X-Y-Z and comprises one or more detectable markers attached to both ends or the inside thereof. When the Y region of the promer is cleaved by a specific enzyme, the Y and Z regions are separated from the template, and the X region serves as a primer without separation from the template. The structure of such promer is as described above.

The above detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

In one specific example, where the promer of the present invention is used as a forward primer and a probe or as a reverse primer and a probe, the promer may comprise a nucleotide sequence capable of complementarily binding to a portion of the nucleotide sequence of the DNA extracted in step (a).

In another specific example, where the promer of the present invention is used as a forward primer and a probe or as a reverse primer and a probe, a separate reverse primer or forward primer may be used in addition to the promer. Herein, the separate reverse primer or forward primer is a DNA that can complementarily bind to the DNA extracted in step (a) and that comprises 5-30 nucleotides, preferably 10-30 nucleotides.

In another specific example, where the promer of the present invention is used as a forward primer, a reverse primer and a probe, the promer comprises a nucleotide sequence capable of complementarily binding to a portion of the nucleotide sequence of the DNA extracted in step (a), and a separate reverse primer or forward primer other than the promer is not required.

In the present invention, amplification of the DNA is preferably performed at an isothermal temperature at which the cDNA is annealed with (A) the promer of the present invention, which is used as a forward primer and a probe or as a reverse primer and a probe, and a forward primer or reverse primer, which is generally used in the art, or (B) the promer of the present invention, which is used as a forward primer, a reverse primer and a probe, and at which the activity of the enzyme used is not substantially inhibited. As used herein, the term "isothermal temperature" means that there is no thermal cycling, and the term does not necessarily mean physically equivalent temperature.

In one specific example, the isothermal temperature at which amplification in the present invention is performed may be 40°C to 80°C, preferably 55°C to 75°C, more preferably 60°C to 75°C.

In the present invention, amplification of the DNA may be performed by one method selected from the group consisting of polymerase chain reaction, rolling circle amplification, strand displacement amplification, and nucleic acid sequence-based amplification, but is not necessarily limited thereto.

In the present invention, amplification of the DNA may be performed using reagents required for amplification, in addition to a kit comprising the promer of the present invention. The reagents required for amplification may include, for example, suitable amounts of DNA polymerase (e.g., thermostable DNA polymerase derived from *Thermus aquatiucs* (Taq), *Thermus thermophilus* (Tth), *Thermus filiformis, Thermis flavus, Thermococcus literalis* or *Phyrococcus furiosis* (Pfu)), DNA polymerase cofactor (Mg²⁺), buffer, dNTPs (dATP, dCTP, dGTP and dTTP) and water (dH₂O). In addition, the buffer may include, but are not limited to, suitable amounts of Triton X-100, dimethylsufoxide (DMSO), Tween 20, nonidet P40, PEG 6000, formamide and bovine serum albumin (BSA).

Step (c) is a step of measuring the amount of fragments of the promer cleaved by the enzyme capable of cleaving the Y region of the promer.

In the present invention, measurement of the amount of fragments of the promer may be performed using various detection methods. Specifically, the amount of fragments of the promer cleaved according to the present invention is preferably measured after completion of RT-PCR or amplification, and may be determined by measuring a change in fluorescence intensity or chemiluminescence.

Measurement of the change in fluorescence intensity or chemiluminescence may be performed using any measurement system capable of detecting a fluorescent label, known in the art. For example, the measurement may be performed using a real-time PCR machine, a TRIAD multimode detector, a Wallac/Victor fluorescence plate reader or a Perkin-Elmer LB50B luminescence spectrometer, LightCycler 96, Applied Biosystems 7500, or Biorad CFX96 real-time PCR thermocycler, but is not limited thereto.

The method for measurement and detection of the amount of fragments of the promer cleaved according to the present invention may vary depending on the kind of label or detectable marker introduced into the promer or a reaction solution.

For example, where the promer, in which a fluorescent label is attached to the end of X region and a quencher is attached to the end of Z region, hybridized with the target nucleic acid, the fluorescence of the fluorescent label attached to the end of X region is greatly reduced by the quencher attached to the end of Z region before the Y and Z regions are cleaved by an enzyme capable of cleaving the Y region. However, when the X region is not separated by the enzyme capable of cleaving the Y region, and only the Y and Z regions are separated from the target nucleic acid and dispersed in the reaction solution, the fluorescence of the fluorescent label attached to the end of X region is increased without being affected by the quencher attached to the end of Z region. At this time, it is possible to detect the target nucleic acid in real time by measuring the increased fluorescence emission of the fluorescent label attached to the end of X region using the above-described apparatus.

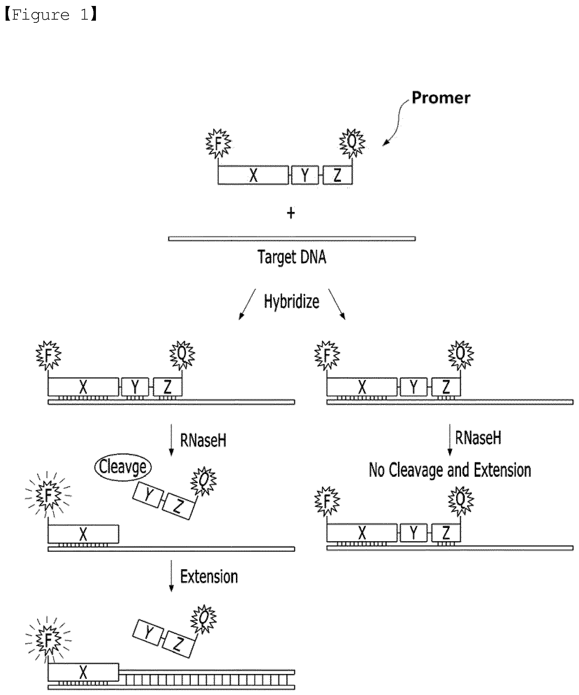

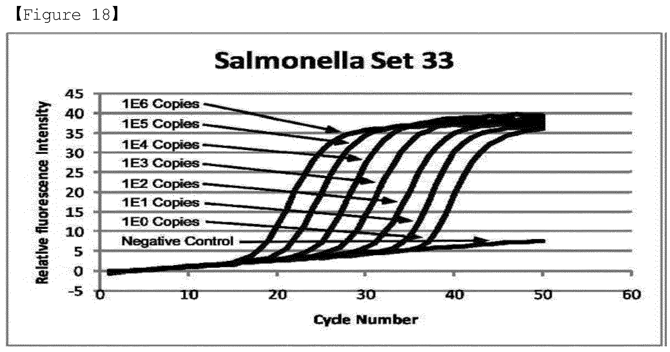

The method for detection of nucleic acid using the promer according to the present invention provides the following improvements over conventional methods.
1. In the conventional method, nucleic acids were detected in real time by preparing and using forward and reverse primers and probes, respectively. However, the present invention can detect nucleic acids in real time by using and preparing one forward or reverse promer, and one forward or reverse primer, or by using and preparing one forward and reverse promer. Therefore, the present invention provides a cost-effective and simple analysis method that uses a small amount of oligo compared to a conventional method for real-time detection of nucleic acid. In particular, conventional Catacleave probe is used only as probes and not as primers, but the promer of the present invention is used simultaneously as primers and probes (see FIG. 1).
2. Conventional method requires a separate probe for real-time detection and requires an amplification interval of at least 60 to 70 bp. However, since the present invention can be simultaneously used as a primer and a probe, the method of the present invention does not require a separate probe for real-time detection, and thus can achieve real-time detection through a shorter amplification span. For example, the prior art analysis method requires an amplification interval of at least 60 bp - 70 bp, but requires an amplification interval of 40 bp or more when the promer of the present invention is used. This provides a more quick and simple detection method when using the present invention, since additional oligo extension is not required in a process for analysis of small nucleic acid analysis such as miRNA. Particularly, in the conventional Catacleave Probe, the cleavage site is cleaved by the cleavage enzyme, and then the front and the rear of the cleavage site is separated from the target nucleic acid and the protein. However, the promer of the present invention can be simultaneously used as a primer and a probe, since X region retains the complex with the target nucleic acid and only the Y and Z regions are separated from the target nucleic acid after the cleavage of Y region.
3. The amplification step following cleavage of the Y region of the promer of the present invention facilitates detection of mutations in the Y region, and thus the promer of the present invention enables the detection of a number of mutations through subsequent nucleic acid amplification. That is, when hybridization is formed between the Y region of the promer of the present invention and the mutation region of target nucleic acid, the Y region is cleaved only when the Y region is exactly complementary to the mutation region of target nucleic acid, and then the amplification reaction is performed. At this time, the mutation of the target nucleic acid can be clearly identified. Specifically, even if the Y region of the promer of the present invention and the mutation region of target nucleic acid are allowed to hybridize, the Y region is not cleaved and the amplification reaction does not occur in case that the Y region does not have a complementary binding. It means that there is no mutation to be detected in the target nucleic acid. In contrast, even if the Y region of the promer of the present invention and the mutation region of target nucleic acid, which is not mutated, are allowed to hybridize, the Y region is not cleaved and the amplification reaction does not occur in case that the Y region does not have a complementary binding. It means that there is no mutation to be detected in the target nucleic acid.
4. In the conventional method, Catacleave Probe represents a trend to be continuously increased in the background fluorescence intensity by the repeated cleavages as shown in FIG. 18, so that it is difficult to accurately measure the Ct value (threshold cycle value) and a separate operation is required to measure the Ct value. However, the present invention does not require a separate operation for measuring the Ct value and enables accurate measurement.
5. The prior hydrolysis probe, such as Catacleave Probe and Taqman probe, does not generate accurate fluorescent indicators as the amount of amplification through one amplification. For example, in the case of TaqMan probes or MGB probes, hybridized probes should be cleaved during the synthesis of oligonucleotides, but they may be seperated without being cleaved. In the case of the Catacleave Probe, it generates more fluorescent indicators than the amount of amplification of the actual target nucleic acid due to repetitive hybridization and cleavage. However, the promer of the present invention hybridizes with a target nucleic acid, and then one fluorescent indicator is generated by Y site cleavage and an oligonucleotide is synthesized. Therefore, the amount of the fluorescent indicator is exactly matched with the target nucleic acid to be amplified.
6. The present invention provides an improved multiplex detection method that uses the promer of the present invention as forward and reverse primers. Although the kind of fluorescent label used for conventional multiplex detection is can be used, a more diverse kind of nucleic acid can be detected using the same or different fluorescent labels attached to the promer of the present invention. Generally, the use of two fluorescent labels enables multiplex detection of five nucleic acids, the use of three fluorescent labels enable multiplex detection of nine nucleic acids, and the use of four fluorescent labels enables multiplex detection of fourteen nucleic acids.

In still another aspect, the present invention provides a method for real-time detection of protein using the promer of the present invention.

The method comprises the steps of:
(a) preparing an antibody having attached thereto a nucleic acid having a nucleotide sequence complementary to the promer of the present invention; (b) binding the antibody, prepared in step (a), to a sample containing a protein to be detected, thereby forming a protein-antibody complex; c) hybridizing a kit comprising the promer of the present invention to the protein-antibody complex formed in step (b), thereby forming a protein-antibody-promer complex; and d) measuring the amount of fragments of the promer cleaved through step (c).

Each step of the method for detection of protein according to the present invention will now be described.

Step (a) is a step of preparing an antibody having attached thereto a nucleic acid having a nucleotide sequence complementary to the promer of the present invention.

In the present invention, the nucleic acid having a nucleotide sequence complementary to the promer of the present invention is synthesized by conventional PCR amplification using the nucleotide sequence of the promer of the present invention as a template.

As mentioned above, the promer of the present invention has a structure of X-Y-Z and comprises one or more detectable markers attached to both ends or the inside thereof. When the Y region of the promer is cleaved by a specific enzyme, the Y and Z regions are separated from the template, and the X region serves as a primer. This promer structure is as described above.

The above detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

In the present invention, the antibody has a structure in which the nucleotide sequence complementary to the promer of the present invention is linked to the Fc region of the antibody by a linker. Herein, the linker is generally a DNA consisting of 1-10 nucleotides.

In the present invention, to link the antibody with the nucleic acid having a nucleotide sequence complementary to the promer of the present invention, two methods may generally be used. In the first method, a DNA having a thiol-modified 5' end is linked to the free amino groups of the antibody by use of one reagent selected from the group consisting of succinimidyl-4-(N-maleimidomethl)cyclohexane-1-carboxylate (SMMCC), Sulfo-succinimidyl-4-(N-maleimidomethl)cyclohexane-1-carboxylate (Sulfo-SMCC), n-succinimidyl-3-(2-pyridylthio)propionate (SPDP), N-succinimidyl-6-(3'-(2-pyridyldithio)-propionamido)hexanoate (NHS-Ic-SPDP), and sulfo-succinimidyl-6-(3'-(2-pyridyldithio)-propionaamido)hexanoate (Sulfo-NHS-Ic-SPDP). Such reagents have different spacer lengths and water solubilities. If necessary, for an additional operation, the linking region can be cleaved using a thiolation reagent that releases DNA.

In the second method, a linking region is provided between the antibody and DNA by the tetrameric protein strepavidin, wherein the protein sufficiently forms an irreversible bond with biotin. The free amino groups of the antibody are labeled with biotin by reaction with biotin-N-hydroxysuccinimide. Biotinylation of the DNA may be performed by the use of 5'-biotin phoshporamidite or the reaction of biotin-n-hydroxysuccinimide following an amino group at the 5'-end. A conjugate of DNA, strepavidin and the antibody can be prepared by adding 1 molar equivalent of a DNA-strepavidin conjugate.

According to the above-described method, the antibody having attached thereto the nucleic acid having the nucleotide sequence complementary to the promer of the present invention may be allowed to react at 4°C for 1 hour, followed by purification with a Superdex 200 gel column, thereby obtaining an antibody-nucleic acid conjugate.

The antibody prepared according to the present invention can specifically recognize the protein to be detected, while the promer of the present invention can hybridize to the antibody. Thus, the antibody can be easily used for real-time detection of the protein to be detected.

Step (b) is a step of forming a protein-antibody complex.

Specifically, step (b) of the method of the present invention is a step of binding the antibody, prepared in step (a), to a sample containing a protein to be detected, thereby forming a protein-antibody complex.

In the present invention, the sample may be a biological sample containing the protein to be detected. Specifically, the sample may be any one or more selected from the group consisting of blood, saliva, urine, feces, tissue, cell and biopsy samples, or may be a stored biological sample containing the protein containing the protein to be detected, but is not necessarily limited thereto.

The stored biological sample may be stored by any conventional method known in the art. The sample may be one stored for more than 1 year, for example, 1 to 10 years. Alternatively, the sample may be one derived from freeze-stored tissue or from formalin-fixed tissue stored at room temperature.

In the present invention, binding of the antibody to the protein may be performed by mixing the antibody prepared in step (a) with a sample containing the protein to be detected.

Step (c) is a step of forming a protein-antibody-promer complex.

Specifically, step (c) of the method of the present invention is a step of hybridizing a kit comprising the promer of the present invention to the protein-antibody complex formed in step (b), thereby forming a protein-antibody-promer complex.

In the present invention, the kit comprising the promer refers to a kit comprising the promer of the present invention and an enzyme capable of cleaving the Y region of the promer. In the present invention, the enzyme capable of cleaving the Y region of the promer may be any enzyme capable of specifically cleaving the Y region of the promer. For example, when the Y region is DNA, the enzyme capable of cleaving the Y region is preferably DNA nuclease (DNase), specifically DNase I, DNase II, S1 nuclease, nuclease P1, AP endonuclease, or UvrABSC nuclease. When the Y region is RNA, the enzyme capable of cleaving the Y region is preferably ribonuclease (RNase), specifically RNase II, RNase III, RNase IV, RNase H, or RNase T₂.

In the present invention, hybridization of the kit comprising the promer of the present invention to the protein-antibody complex may be performed by adding the kit comprising the promer of the present invention to the protein-antibody complex.

Step (d) is a step of detecting the protein by measuring the amount of fragments of the promer cleaved by the enzyme capable of cleaving the Y region of the promer.

In the present invention, measurement of the amount of fragments of the promer may be performed using various detection methods. Specifically, the amount of fragments of the promer cleaved according to the present invention is preferably measured after completion of RT-PCR or amplification, and may be determined by measuring a change in fluorescence intensity or chemiluminescence.

Measurement of the change in fluorescence intensity or chemiluminescence may be performed using any measurement system capable of detecting a fluorescent label, known in the art. For example, the measurement may be performed using a real-time PCR machine, a TRIAD multimode detector, a Wallac/Victor fluorescence plate reader, a Perkin-Elmer LB50B luminescence spectrometer, LightCycler96, Applied Biosystems 7500, or Biorad CFX96 real-time PCR thermocycler, but is not limited thereto.

The method for measurement and detection of the amount of fragments of the promer cleaved according to the present invention may vary depending on the kind of label or detectable marker introduced into the promer or a reaction solution.

For example, a promer, wherein a fluorescent label is attached at the end of X region and a quencher is attached at the end of the Z region, is hybridized with an antibody that forms a complex with a protein to be detected, and fluorescence of the fluorescent label attached at the end of X region is greatly reduced by the quencher attached to the end of Z region before the Y and Z regions are separated by an enzyme capable of cleaving the Y region. However, when the X region is not separated but only the Y and Z regions are separated from the antibody and dispersed in the reaction solution by the enzyme capable of cleaving the Y region, the fluorescence of the fluorescent label attached to the end of X region is increased without being influenced by to the quencher attached to the end of Z region. At this time, it is possible to detect the target protein in real time by using the above-described apparatus for increasing fluorescence emission of the fluorescent label attached at the end of X region.

As one example, the above detectable marker may be either a fluorescent label that binds to the promer by covalent binding or non-covalent binding, or a fluorescent pair of the fluorescent label and a quencher.

The fluorescent label may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670 and biotin, but is not necessarily limited thereto. Furthermore, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2 and MGBNFQ, but is not necessarily limited thereto.

The method for detecting protein according to the present invention has an advantage in that the detection speed and detection accuracy of the protein to be detected can be increased by using the promer of the present invention and an antibody having attached thereto a nucleic acid having a nucleotide sequence complementary to the promer of the present invention. That is, as described above, the present invention provides faster detection speed and accuracy of a promer bound to a protein-antibody complex by a short amplification period and high specificity.

### [Advantageous Effects]

As described above, the method for detection of nucleic acid (DNA or RNA) or protein using the promer according to the present invention uses a small amount of oligo compared to a conventional detection method and does not require a separate probe for real-time detection. Thus, according to the method of the present invention, the nucleic acid (DNA or RNA) or protein to be detected can be detected in real time in a cost-effective and simple manner.

Furthermore, mutations in the Y region can be detected through the amplification step after cleavage of the Y region of the promer, and multiplex detection of nucleic acids (DNA or RNA) or proteins larger than the number of fluorescent labels attached to the promer is possible.

Thus, the promer of the present invention and the method for real-time detection of nucleic acid (DNA or RNA) or protein using the promer can distinguish various point mutations occurring in KRAS, BRAF, EGFR, etc., and thus can be advantageously used for diagnosis of various diseases and for diagnosis of prognosis and can be effectively used for various bacteria, such as antibiotic resistant bacteria occurring in point mutation, and viruses.

### [Description of Drawings]

FIG. 1 illustrates a process of detecting a target nucleic acid using a promer according to the present invention.
FIG. 2 shows the results of a polymerase chain reaction performed using the primers of SEQ ID NOs: 2 and 7, prepared in an example of the present invention, to measure expression of CK-18 gene in a SW620 human cell line and PBMC (a peripheral blood mononuclear cell).
FIG. 3 shows the results of a polymerase chain reaction performed using the primers of SEQ ID NOs: 3 and 8, prepared in an example of the present invention, to measure expression of CK-19 gene in a SW620 human cell line and PBMC.
FIG. 4 shows the results of a polymerase chain reaction performed using the primers of SEQ ID NOs: 6 and 9, prepared in an example of the present invention, to measure expression of GAPDH gene in a SW620 human cell line and PBMC.
FIG. 5 shows the results of a polymerase chain reaction performed using the promer of SEQ ID NO: 1 and the reverse primer of SEQ ID NO: 2, prepared in an example of the present invention, to measure expression of CK-18 gene in a SW620 human cell line and PBMC.
FIG. 6 shows the results of a polymerase chain reaction performed using the forward primer of SEQ ID NO: 3 and the promer of SEQ ID NO: 4, prepared in an example of the present invention, to measure expression of CK-19 gene in a SW620 human cell line and PBMC.
FIG. 7 shows the results of a polymerase chain reaction performed using the promer of SEQ ID NO: 5 and the reverse primer of SEQ ID NO: 6, prepared in an example of the present invention, to measure expression of GAPDH gene in a SW620 human cell line and PBMC.
FIG. 8 shows the results of a polymerase chain reaction performed using the promer of SEQ ID NO: 11 and the reverse primer of SEQ ID NO: 12, prepared in an example of the present invention, to measure G12D mutant in KRAS gene.
FIG. 9 shows the results of a polymerase chain reaction performed using the promer of SEQ ID NO: 10 and the reverse primer of SEQ ID NO: 12, prepared in an example of the present invention, to measure G12V mutant in KRAS gene.
FIG. 10 shows the results of a polymerase chain reaction performed using the promer of SEQ ID NO: 10 and the reverse primer of SEQ ID NO: 12, prepared in an example of the present invention, to measure G12D mutant in KRAS gene.
FIG. 11 shows the results of detecting T529C mutant in OPN1MW gene after performing a polymer chain reaction using the promer of SEQ ID NO: 14 and the reverse primer of SEQ ID NO: 19, prepared in an example of the present invention.
FIG. 12 shows the results of detecting T529C mutant in OPN1MW gene after performing a polymer chain reaction using the promer of SEQ ID NOs: 14 and 15 and the reverse primer of SEQ ID NO: 19, prepared in an example of the present invention.
FIG. 13 shows the results of detecting T529C mutant in OPN1MW gene after performing a polymer chain reaction using the promers of SEQ ID NOs: 14 and 16 and the reverse primer of SEQ ID NO: 19, prepared in an example of the present invention.
FIG. 14 shows the results of detecting T529C mutant in OPN1MW gene after performing a polymer chain reaction using the promers of SEQ ID NOs: 14 and 17 and the reverse primer of SEQ ID NO: 19, prepared in an example of the present invention.
FIG. 15 shows the results of detecting T529C mutant in OPN1MW gene after performing a polymer chain reaction using the promers of SEQ ID NOs: 14 and 18 and the reverse primer of SEQ ID NO: 19, prepared in an example of the present invention.
FIG. 16 shows the results of detecting G12D mutant in KRAS gene after performing a polymer chain reaction using the promers of SEQ ID NOs: 20 and 21 and the reverse primer of SEQ ID NO: 12, prepared in an example of the present invention.
FIG. 17 shows the results of detecting G12V mutant in KRAS gene after performing a polymer chain reaction using the promers of SEQ ID NOs: 22 and 23 and the reverse primer of SEQ ID NO: 12, prepared in an example of the present invention.
FIG. 18 show the results of confirming whether or not the Salmonella Set 33 was amplified using Catacleave probe according to the prior art.

### [Mode for Invention]

Hereinafter, the present invention will be described in further detail with reference to examples. It will be obvious to those skilled in the art that these examples are illustrative purposes only and are not intended to limit the scope of the present invention.

### Example 1: Real-Time Analysis of Nucleic Acid Using Promer of the Present Invention

In order to measure the expression of CK-18 (cytokeratin-18), CK-19 (cytokeratin-19) and GAPDH (glyceraldehyde 3-phophate dehydrogenase) genes, the promers according to the present invention and primers were constructed by IDT (Integrated DNA Technologies, USA) as shown in Table 1 below (see Table 1). Herein, for the promers, FAM (fluorescein succinimidyl ester) was attached to the 5' end, and 3IABkFG was attached to the 3' end. For ribonucleic acid (RNA), the letter "r" was added to the front of the sequence for discrimination from deoxyribonucleic acid (DNA). As controls, the primers to be used in the SYBR method were prepared by IDT as shown in Table 2 below.

**Table 1**

| | | |
|---|---|---|
| Human CK18 F-Promer | 5'-ATCTTGGTGATGCCTTrGrGAC-3' | SEQ ID NO: 1 |
| Human CK18 R primer | 5'-CCTGCTTCTGCTGGCTTAAT-3' | SEQ ID NO: 2 |
| Human CK19 F primer | 5'-GTCACAGCTGAGCATGAAAG -3' | SEQ ID NO: 3 |
| Human CK19 R-Promer | 5'-TCACTATCAGCTCGCArCrATC-3' | SEQ ID NO: 4 |
| Human GAPDH F-Promer | 5'-AAGGTGAAGGTCGGAGrUrCAA-3' | SEQ ID NO: 5 |
| Human GAPDH R primer | 5'-AATGAAGGGGTCATTGATGG-3' | SEQ ID NO: 6 |

**Table 2**

| | | |
|---|---|---|
| Human CK18 F primer | 5'-ATCTTGGTGATGCCTTGGAC-3' | SEQ ID NO: 7 |
| Human CK18 R primer | 5'-CCTGCTTCTGCTGGCTTAAT-3' | SEQ ID NO: 2 |
| Human CK19 F primer | 5'-GTCACAGCTGAGCATGAAAG-3' | SEQ ID NO: 3 |
| Human CK19 R primer | 5'-TCACTATCAGCTCGCACATC-3' | SEQ ID NO: 8 |
| Human GAPDH F primer | 5'-AAGGTGAAGGTCGGAGTCAA-3' | SEQ ID NO: 9 |
| Human GAPDH R primer | 5'-AATGAAGGGGTCATTGATGG-3' | SEQ ID NO: 6 |

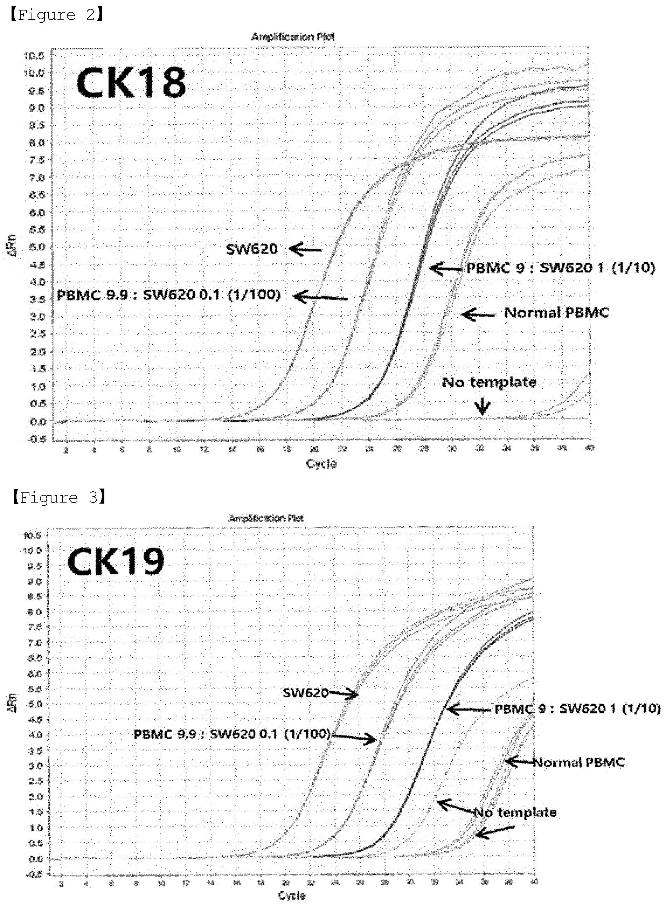

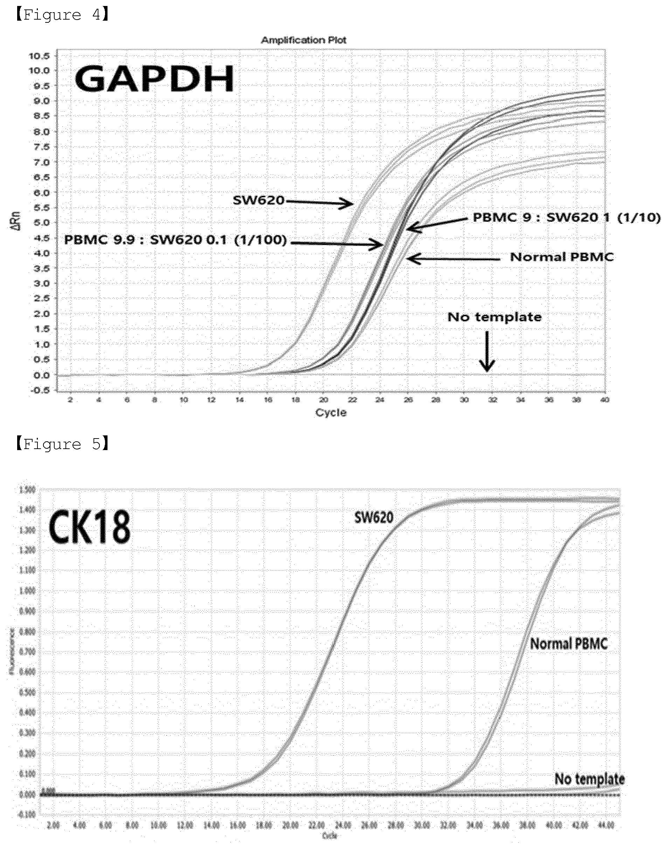

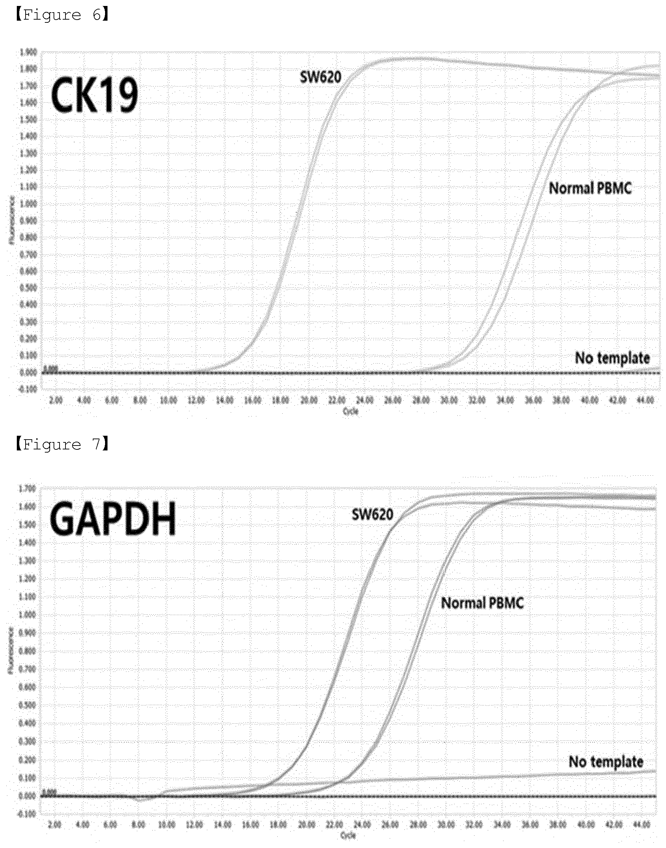

RNA from each of the SW620 and PMBC obtained from human blood was subjected to reverse transcription polymerase chain reaction (RT-PCR) to synthesize cDNAs. 10 ng of each cDNA, 10m units of heat-resistant RNase H and 4 µl of AptaTaq DNA master (Roche) were added to a tube, and the total volume was adjusted to 20 µl using triple distilled water. Next, the cDNA was subjected to a polymerase chain reaction (PCR) was performed in the presence of 1 µl of 10 µM concentration of each promer and each primer, shown in Tables 1 and 2. Herein, the PCR reaction was performed for 40 cycles (SYBR method) and 45 cycles (the promer of the present invention), each cycle consisting of 5 min at 95°C, 60 secs at 62 to 63°C and 10 sec at 95°C. The results of the PCR are shown in FIGS. 2 to 7.

As a result, it could be seen that, when expression of CK-18, CK-19 and GAPDH was measured using the SYBR method, a nonspecific amplification curve for CK-18 and CK-19 excluding GAPDH appeared in the negative control, and nonspecific amplification also occurred in the positive control (see FIGS. 2 to 4). On the contrary, when the promer according to the present invention was used, problems such as nonspecific amplification did not occur in the case of not only GAPDH but also CK-18 and CK-19. Thus, it can be seen that the promer according to the present invention can be used in gene expression and analysis without causing any nonspecific reaction, unlike the conventional SYBR method.

### Example 2: Measurement of Mutant Using the Promer of the Present Invention

Using the promer of the present invention, G12D and G12V mutants in KRAS gene were measured. Specifically, the promers according to the present invention and a reverse primer were constructed by IDI as shown in Table 3 below. For the promers, FAM (fluorescein succinimidyl ester) was attached to the 5' end, and 3IABkFG was attached to the 3' end. For ribonucleic acid (RNA), the letter "r" was added to the front of the sequence for discrimination from deoxyribonucleic acid (DNA).

**Table 3**

| | | |
|---|---|---|
| G12D Forward-Promer | 5'-CTTGTGGTAGTTGGAGCTGrATG-3' | SEQ ID NO: 10 |
| G12V Forward-Promer | 5'-ACTTGTGGTAGTTGGAGCTGrATG-3' | SEQ ID NO: 11 |
| Uni-reverse primer | 5'-CATATTCGTCCACAAAATGATTCTG-3' | SEQ ID NO: 12 |

In addition, the KRAS gene to be measured was obtained as total DNA from each of SW620 cell line and LS174T cell line. For wild-type gene, total DNA was obtained from the blood of normal persons in the same manner. Herein, SW620 is known as G12V mutant cell-line, and LS174T is known as G12D mutant cell-line.

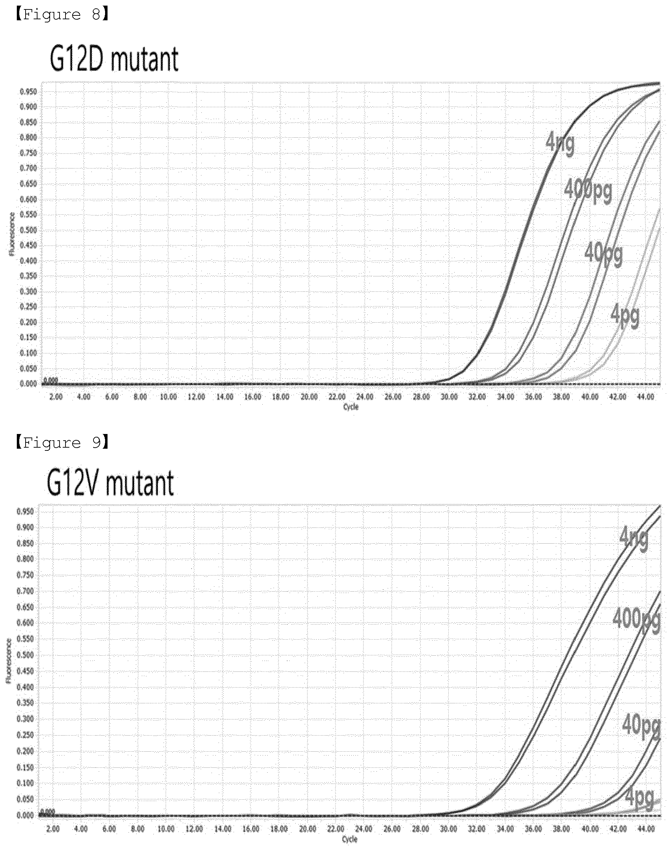

Next, 10 m units of heat-resistant RNase H, 4 µl of AptaTaq DNA Master (Roche), 40 ng of total DNA extracted from SW620, and each of 4 pg, 40 pg, 400 pg, and 4 ng of total DNA extracted from LS174T were placed in a tube, and then the total volume was adjusted to 20 µl using triple distilled water. Thereafter, 1 µl of 10 µM concentration of the promer of SEQ ID NO: 11 and the primer of SEQ ID No: 12, constructed as described above, were prepared, and then polymerase chain reaction was performed to measure G12D mutant known as LS174T mutant. Herein, the PCR reaction was performed under the conditions of 5 min at 95°C, 60 secs at 62 to 63°C and 10 secs at 95°C. The results of the measurement are shown in FIG. 8.

In addition, G12V mutant known as SW620 mutant was measured in the presence of the promer of SEQ ID NO: 10 and the primer of SEQ ID NO: 12 under the same conditions as described above, except that each of 4 pg, 40 pg, 400 pg, and 4 ng of total DNA extracted from SW620 was added to 40 ng of total DNA extracted from LS174T, instead of adding total DNA extracted from SW620 to total DNA extracted from LS174T. The results of the measurement are shown in FIG. 9.

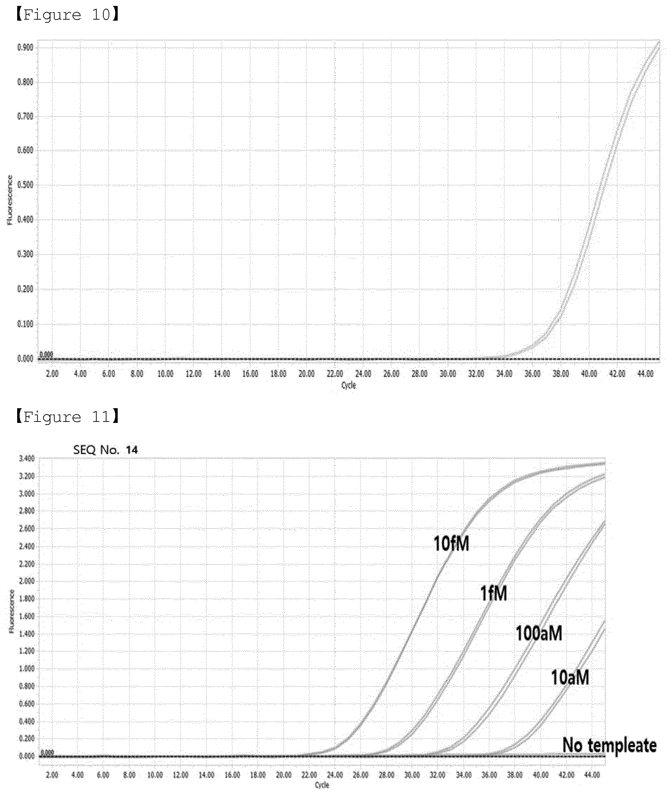

In addition, G12D mutant known as LS174T mutant was measured in the presence of the promer of SEQ ID NO: 10 and the primer of SEQ ID NO: 12 under the same conditions as described above, except that 40 pg of total DNA extracted from LS174T cell line was added to 40 ng of total RNA from normal persons, instead of adding total DNA extracted from SW620 to total DNA extracted from LS174T. The results of the measurement are shown in FIG. 10.

From the above results, it can be seen that, when the promer according to the present invention is used, a mutant can be measured with excellent sensitivity and specificity.

Namely, in the endpoint genotyping, which is widely used as a method to measure point mutations, only the presence or absence of mutation can be identified through real-time PCR reaction. Respectively. However, in the case of using the promer according to the present invention, it was found that the type and the ratio of the mutation can be analyzed through the real-time PCR reaction as described above. Furthermore, in the case of the conventional method, a maximum of 1 ∼ 0.1% mutation can be confirmed, but in the case of the present invention, 0.01% mutation analysis is possible.

### Example 3: Determination of Optimal Size of the Promer According to the Present invention

The OPN1MW gene (medium-wave-sensitive opsin-1 gene) located in the long arm (Xq28) of chromosome X is a gene associated with color weakness and color blindness, and it is known that mutations occur at four nucleotide locations of the gene to cause red-green color blindness. The positions of the mutations are C282A, T529C, T607C and G989A.

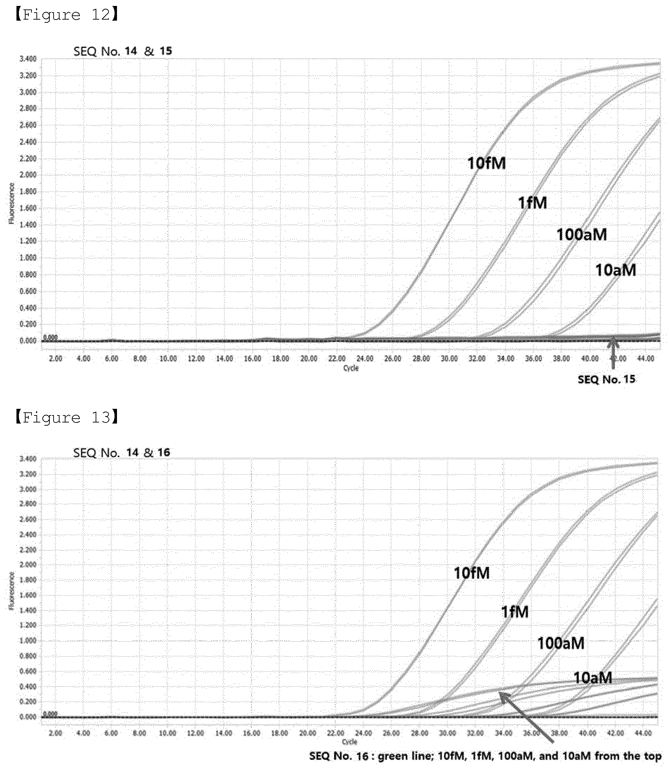

Accordingly, in order to examine the mutant detection ability of the promer of the present invention according to the length thereof, a plasmid containing a gene (see the DNA sequence of SEQ ID NO: 13) obtained by mutating the T529C position of the OPN1MW gene was constructed, and then diluted to concentrations of 10 fM, 1 fM, 100 aM and 10 aM. 1 µl of each of the dilutions was taken, and then five promers and a reverse primer were constructed from each dilution by IDT as shown in Table 4 below. The constructed promers and primer were used for detection. Herein, FAM (fluorescein succinimidyl ester) was attached to the 5' end of the five constructed promers, and 3IABkFG was attached to the 3' end. For ribonucleic acid (RNA), the letter "r" was added to the front of the sequence for discrimination from deoxyribonucleic acid (DNA). The results obtained using the promer of SEQ ID NO: 14 (see FIG. 11) and the results obtained using the promer of SEQ ID NO: 13 and each of the promers of SEQ ID NOs: 15, 16, 17, and 18 (see FIGS. 12 to 15) are shown in FIGS. 11 to 15.

**Table 14**

| | | |
|---|---|---|
| 529CA1 Promer | 5'-TGGGCATTGCCTTCTCCrCGG-3' | SEQ ID NO: 14 |
| 529CA2 Promer | 5'-GCCTTCTCCrCGG-3' | SEQ ID NO: 15 |
| 529CA3 Promer | 5'-CAAGCTGGCCATCGTGGGCATTGCCTTCTCCrCGG-3' | SEQ ID NO: 16 |
| 529CA4 Promer | 5'-TGGGCATTGCCTTCTCCrCGGATCTGGGC-3' | SEQ ID NO: 17 |
| 529CA5 Promer | 5'-TGGGCATTGCCTTCTCCrCGGATCTGGGCTGCTGTG-3' | SEQ ID NO: 18 |
| Reverse primer | 5'-GTACCTGCTCCAACCAAAGA-3' | SEQ ID NO: 19 |

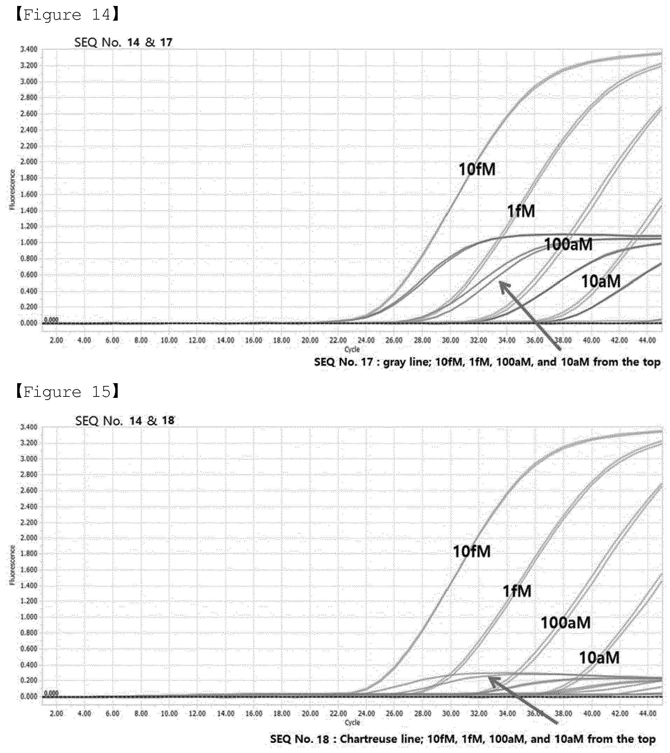

As can be seen in FIGS. 11 to 15, when the X region in the X-Y-Z structure of the promer according to the present invention consisted of 9 nucleotides, the X region was also separated from the template after cleavage of the Y region, and thus served only as a probe without serving as a primer (see FIG. 12), and when the X region consisted of 31 nucleotides, a nonspecific amplification reaction was observed (see FIG. 13). Furthermore, when the Z region consisted of 2 or 10 nucleotides, only the Z region was separated from the template after cleavage of the Y region, and the X region was not separated, and thus a normal amplification reaction occurred (see FIGS. 11 and 14), but when the Z region consisted of 17 nucleotides, the Z region was not separated from the template after cleavage of the Y region, and thus a normal amplification reaction did not occur (see FIG. 15). As described above, it could be seen that, when the X and Z regions of the promer according to the present invention consisted of a specific number of nucleotides, a specific amplification reaction could occur.

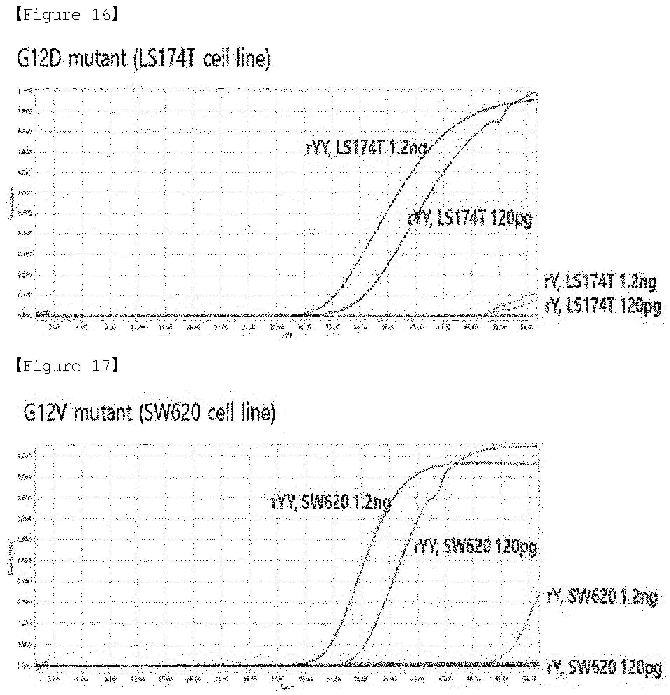

In addition, a mutant in KRAS gene was measured in the same manner as the experiment of Example 2, except that, as shown in Table 5 below, the Y region consisted of one RNA and the Z region consisted of one or two DNAs. Specifically, 10 µM of the promer of SEQ ID NO: 20 and 1 µl of the primer of SEQ ID NO: 10, or 10 µM of the promer of SEQ ID NO: 21 and 1 µl of the primer of SEQ ID NO: 12, 10m units of heat-resistant RNase H, 4 µl of AptaTaq DNA master (Roche), and each of 120 pg and 1.2 ng of total DNA extracted from LS174T were placed in a test tube, and the total volume was adjusted to 20 µl using triple distilled water. Next, a polymerase chain reaction was performed under the conditions of 5 min at 95°C, 60 sec at 62°C to 63°C and 10 sec at 95°C, thereby measuring G12D mutant known as LS174T mutant. The results of the measurement are shown in FIG. 16.

In addition, 10 µM of the promer of SEQ ID NO: 22 and 1 µl of the primer of SEQ ID NO: 12, or 10 µM of the promer of SEQ ID NO: 23 and 1 µl of the primer of SEQ ID NO: 12, 10m units of heat-resistant RNase H, 4 µl of AptaTaq DNA master (Roche), and each of 120 pg and 1.2 ng of total DNA extracted from SW620 were placed in a test tube, and the total volume was adjusted to 20 µl using triple distilled water. Next, a polymerase chain reaction was performed under the conditions of 5 min at 95°C, 60 sec at 62°C to 63°C and 10 sec at 95°C, thereby measuring G12V mutant known as SW620 mutant. The results of the measurement are shown in FIG. 17.

**Table 5**

| | | |
|---|---|---|
| G12D Forward-Promer rY Type | 5'-ACTTGTGGTAGTTGGAGCTGrAT-3' | SEQ ID NO: 20 |
| G12D Forward-Promer rYY Type | 5'-CTTGTGGTAGTTGGAGCTGrATG-3' | SEQ ID NO: 21 |
| G12V Forward-Promer rY Type | 5'-AACTTGTGGTAGTTGGAGCTGrUT-3' | SEQ ID NO: 22 |
| G12V Forward-Promer rYY Type | 5'-ACTTGTGGTAGTTGGAGCTGrUTG-3' | SEQ ID NO: 23 |
| Uni-reverse primer | 5'-CATATTCGTCCACAAAATGATTCTG-3' | SEQ ID NO: 12 |

As can be seen in FIGS. 16 and 17, when the spacing between the Y region to be cleaved and the Z region was at least 2 bp, the amplification reaction using the promer of the present invention smoothly occurred.

## Claims

1. A promer which is used as a primer and a probe for real-time detection of nucleic acid or protein, has a structure of X-Y-Z, comprises at least one detectable marker attached to both ends or an inside thereof, and forms a complex by binding to a target nucleic acid or a specific region of a target protein to be detected in real time,
wherein when Y region of the promer is cleaved by a specific enzyme, X region act as primer in maintaining a complex with the target nucleic acid or the target protein and Y and Z regions are separated from the specific region of the target nucleic acid or protein, wherein when any one of X, Y and Z is DNA, one or more of the other two are RNA,
wherein X, Y and Z are a DNA or RNA consisting of nucleotides, and
wherein when any one of X, Y and Z is DNA, one or more of the other two are RNA.

2. The promer of claim 1, wherein X is a DNA or RNA consisting of 1 to 60 nucleotides, Y is a DNA or RNA consisting of 1 to 10 nucleotides, Z is a DNA or RNA consisting of 0 to 10 nucleotides, and when Z is null, Y is a DNA or RNA consisting of 3 to 10 nucleotides, and when Z is 1, Y is a DNA or RNA consisting of 2 to 10 nucleotides.

3. The promer of claim 1, wherein X, Y and Z are DNA, RNA and DNA, respectively, or are RNA, DNA and RNA, respectively.

4. The promer of claim 1, wherein the detectable marker is either a fluorescent label that binds to the promer by covalent binding or non-covalent binding, or a fluorescent pair of the fluorescent label and a quencher.

5. The promer of claim 1, wherein X, Y and Z of the promer are synthesized so as to be wholly or partially methylated to prevent nonspecific cleavage.

6. The promer of claim 1, wherein X of the promer is a DNA or RNA consisting of 10 to 30 nucleotides, Y is a DNA or RNA consisting of 1 to 10 nucleotides, and Z is a DNA or RNA consisting of 2 to 10 nucleotides.

7. The promer of claim 1, wherein when the Y region of the promer is DNA, the enzyme is DNA nuclease (DNase), specifically DNase I, DNase II, S1 nuclease, nuclease P1, AP endonuclease, or UvrABSC nuclease, and when the Y region is RNA, the enzyme is ribonuclease (RNase), specifically RNase II, RNase III, RNase IV, RNase H, or RNase T₂.

8. The promer of claim 1, wherein the promer is used as: i) an RT primer for synthesizing cDNA from RNA among nucleic acids; or ii) a forward primer for amplifying cDNA synthesized from nucleic acid (DNA or RNA); or iii) a reverse primer for amplifying cDNA synthesized from nucleic acid (DNA or RNA); or iv) forward and reverse primers for cDNA synthesized from nucleic acid (DNA or RNA); or v) a probe for real-time detection of a nucleic acid (DNA or RNA) or protein to be detected.

9. A kit for detection of nucleic acid, comprising: the promer of any one of claims 1 to 8; and an enzyme capable of cleaving the Y region of the promer.

10. The kit of claim 9, wherein when the Y region of the promer is DNA, the enzyme is DNA nuclease (DNase), specifically DNase I, DNase II, S1 nuclease, nuclease P1, AP endonuclease, or UvrABSC nuclease, and when the Y region is RNA, the enzyme is ribonuclease (RNase), specifically RNase II, RNase III, RNase IV, RNase H, or RNase T₂.

11. The kit of claim 9, further comprising reagents required for amplification of DNA.

12. A method for real-time detection of RNA, comprising the steps of:
(a) extracting RNA from a sample;
(b) adding an RT primer or the promer of any one of claims 1 to 8 to the RNA extracted in step (a), thereby synthesizing cDNA;
(c) adding, to the cDNA synthesized in step (b), (i) the kit of any one of claims 10 to 12, wherein the promer contained in the kit has a nucleotide sequence capable of complementarily binding to a portion of a nucleotide sequence of the cDNA synthesized in step (b) and serves as a forward primer and a probe, and a reverse primer having a nucleotide sequence capable of complementarily binding to a portion of a nucleotide sequence of the cDNA synthesized in step (b); or (ii) the kit of any one of claims 10 to 12 wherein the promer contained in the kit has a nucleotide sequence capable of complementarily binding to a portion of a nucleotide sequence of the cDNA synthesized in step (b) and serves as a reverse primer and a probe, and a forward primer having a nucleotide sequence capable of complementarily binding to a portion of a nucleotide sequence of the cDNA synthesized in step (b); or (iii) the kit of any one of claims 10 to 12, wherein the promer contained in the kit has a nucleotide sequence capable of complementarily binding to a portion of a nucleotide sequence of the cDNA synthesized in step (b) and serves as a forward primer, a reverse primer and a probe, and amplifying the cDNA by extension; and
(d) measuring an amount of fragments of the promer cleaved through step (c).

13. The method of claim 12, wherein step (c) is a step in which the Y region of the promer bound to the cDNA synthesized in step (b) is cleaved by the enzyme contained in the kit so that the Y and Z regions are separated from the cDNA and the X region serves as the primer for amplification.

14. A method for real-time detection of DNA, comprising the steps of:
(a) extracting DNA from a sample;
(b) adding, to the DNA extracted in step (a), (i) the kit of any one of claims 10 to 12, wherein the promer contained in the kit has a nucleotide sequence capable of complementarily binding to a portion of a nucleotide sequence of the DNA extracted in step (a) and serves as a forward primer and a probe, and a reverse primer having a nucleotide sequence capable of complementarily binding to a portion of a nucleotide sequence of the DNA synthesized in step (a); or (ii) the kit of any one of claims 10 to 12, wherein the promer contained in the kit has a nucleotide sequence capable of complementarily binding to a portion of a nucleotide sequence of the DNA extracted in step (a) and serves as a reverse primer and a probe, and a forward primer having a nucleotide sequence capable of complementarily binding to a portion of a nucleotide sequence of the DNA extracted in step (a); or (iii) the kit of any one of claims 10 to 12, wherein the promer contained in the kit has a nucleotide sequence capable of complementarily binding to a portion of a nucleotide sequence of the DNA extracted in step (a) and serves as a forward primer, a reverse primer and a probe, and amplifying the DNA by extension; and
(c) measuring an amount of fragments of the promer cleaved through step (b).

15. The method of claim 14, wherein step (b) is a step in which the Y region of the promer bound to the DNA extracted in step (a) is cleaved by the enzyme contained in the kit so that the Y and Z regions are separated from the cDNA and the X region serves as the primer for amplification.

16. A method for real-time detection of protein in a sample, the method comprising the steps of:
(a) preparing an antibody having attached thereto a nucleic acid having a nucleotide sequence complementary to the promer of any one of claims 1 to 8;
(b) binding the antibody, prepared in step (a), to a sample containing a protein to be detected, thereby forming a protein-antibody complex;
(c) hybridizing the kit of any one of claims 10 to 12 to the protein-antibody complex of step (b), thereby forming a protein-antibody-promer complex; and
(d) measuring an amount of fragments of the promer cleaved through step (c).

17. The method of claim 16, wherein step (d) is a step in which the Y region of the promer bound to the antibody prepared in step (a) is cleaved by the enzyme contained in the kit so that the Y and Z regions are separated from the cDNA and the X region serves as the primer for amplification.
